# EUROPÄISCHE PATENTSCHRIFT

(11) **EP 2 285 768 B1**
(45) Veröffentlichungstag und Bekanntmachung des Hinweises auf die Patenterteilung: **19.03.2014**
(21) Anmeldenummer: 09761412.7
(22) Anmeldetag: 03.06.2009
(51) Int. Cl.: C07C 209/36, B01J 21/18, B01J 27/24, B82Y 30/00

(54) **VERFAHREN ZUR HYDRIERUNG VON ORGANISCHEN VERBINDUNGEN**
PROCESS FOR HYDROGENATING ORGANIC COMPOUNDS
PROCÉDÉ D'HYDROGÉNATION DE COMPOSÉS ORGANIQUES

(30) Priorität: 12.06.2008 DE 102008028070
(43) Veröffentlichungstag der Anmeldung: 23.02.2011
(73) Patentinhaber: Bayer Intellectual Property GmbH, 40789 Monheim (DE)
(72) Erfinder: WOLF, Aurel, 42489 Wülfrath (DE); MICHELE, Volker, 51065 Köln (DE); ASSMANN, Jens, 40723 Hilden (DE); MLECZKO, Leslaw, 41542 Dormagen (DE)
(74) Vertreter: BIP Patents
(86) Internationale Anmeldenummer: PCT/EP2009/003950
(87) Internationale Veröffentlichungsnummer: WO 2009/149849

(56) Entgegenhaltungen:
- US-A1- 2006 142 148
- VAN DOMMELE, S. ET AL: "Nitrogen-containing carbon nanotubes as solid base catalysts" CHEM. COMMUN., 2006, Seiten 4859-4861, XP002544138
- VAN DOMMELE ET AL: "Tuning nitrogen functionalities in catalytically grown nitrogen-containing carbon nanotubes" CARBON, ELSEVIER, OXFORD, GB, Bd. 46, Nr. 1, 7. November 2007 (2007-11-07), Seiten 138-148, XP022442518 ISSN: 0008-6223
- SHALAGINA ET AL: "Synthesis of nitrogen-containing carbon nanofibers by catalytic decomposition of ethylene/ammonia mixture" CARBON, ELSEVIER, OXFORD, GB, Bd. 45, Nr. 9, 19. Juli 2007 (2007-07-19), Seiten 1808-1820, XP022157996 ISSN: 0008-6223
- MATTER ET AL: "Oxygen reduction reaction activity and surface properties of nanostructured nitrogen-containing carbon" JOURNAL OF MOLECULAR CATALYSIS. A, CHEMICAL, ELSEVIER, AMSTERDAM, NL, Bd. 264, Nr. 1-2, 16. Februar 2007 (2007-02-16), Seiten 73-81, XP005893149 ISSN: 1381-1169 in der Anmeldung erwähnt
- LI C-H ET AL: "Nitrobenzene hydrogenation with carbon nanotube-supported platinum catalyst under mild conditions" JOURNAL OF MOLECULAR CATALYSIS. A, CHEMICAL, ELSEVIER, AMSTERDAM, NL, Bd. 226, Nr. 1, 1. Februar 2005 (2005-02-01), Seiten 101-105, XP004692174 ISSN: 1381-1169 in der Anmeldung erwähnt

## Beschreibung

Die Erfindung betrifft einen Katalysator und ein Verfahren zur Hydrierung von organischen Verbindungen.

Hydrierungen sind eine in der chemischen Industrie oft angewandte Umwandlungsmethode für organische Verbindungen mit funktionellen Gruppen wie bspw. Nitro, C-C-Doppelbindung, Carbonyl usw., wobei die entsprechenden Hydrierprodukte (Amine, Alkane, Alkohole usw.) gebildet werden. Die Hydrierung wird im allgemeinem an geträgerten Edelmetallkatalysatoren (Au, Pd, Pt, Ru usw.) oder an sogenanntem Raney-Nickel durchgeführt (S. Nishimura, Handbook of Heterogeneous Catalytic Hydrogenation for Organic Synthesis, 2001, 2-38).

Die auf Oxiden (Silica, Alumina, Titania) oder Aktiv-Kohle geträgerten Edelmetallkatalysatoren sind aufgrund der Edelmetalle sehr teuer und unterliegen einer Desaktivierung durch Versinterung. Raney-Ni Katalysatoren dagegen sind günstiger in der Anschaffung, jedoch sind diese deutlich reaktionsträger als die geträgerten Edelmetallkatalysatoren. Um diesen Nachteil aufzuheben, werden Raney-Ni Katalysatoren oft als feinteiliges Pulver (100-300 µm) eingesetzt, was anderseits zu einer zeitintensiven Abtrennung des Katalysators aus dem Reaktionsgemisch führt. Zusätzlich sind diese Katalysatoren an der Luft pyrophor, so dass deren Handling sicherheitstechnisch stets aufwendig ist.

Es sind daher Verfahren entwickelt worden, die unter Verwendung von sogenannten Kohlenstoffnanoröhrchen den notwendigen Anteil teurer Edelmetalle verringern können. So offenbaren Li et. al. und Zhao et. al. neuartige auf Kohlenstoffnanoröhrchen geträgerte Pt-Katalysatoren für die Hydrierung von Nitrobenzol (J. Molec. Cat. A: chem, 2005, 226(1), 101-105 bzw. Mater. Chem. and Phys., 2007, 103(2-3), 225-229). In beiden Fällen, wird erklärt, dass der katalytische Effekt auf das auf den Kohlenstoffnanoröhrchen aufgebrachte Platin zurückzuführen sei. Die Kohlenstoffnanoröhrchen als solche zeigen keine signifikante katalytische Aktivität. In Mater. Chem. and Phys., 2007, 103(2-3), 225-229 wird weiter erklärt, dass die vorgestellten Katalysatoren durch die Verwendung der Metalle teuer seien. Es wird in beiden Fällen jedoch nicht offenbart, dass die verwendeten Kohlenstoffnanoröhrchen durch Heteroatome modifiziert worden sind, oder dass sich hierdurch ein vorteilhafter Effekt erzielen ließe.

Ein ähnlicher Ansatz wird von Han et. al. (J. Molec. Cat. A: Chem, 2007, 277(1-2), 210-214 und Ind. J. of Chem., Sect A., 2007, 46A(11), 1747-1752) offenbart, der für die Hydrierung von p-/o- und m-Chlorornitrobenzol geeignete bimetallische Katalysatoren auf Oberflächen von Kohlenstoffnanoröhrchen verwendet. Bimetallisch bezeichnet dort das Vorhandensein von weiteren Metallen neben Platin. Insbesondere die Verwendung von bimetallischen Katalysatoren enthaltend Platin, Kohlenstoffnanoröhrchen und Metallen ausgewählt aus der enthaltend Liste Mangan, Eisen, Kobalt, Nickel und Kupfer, wird offenbart. Han et al. offenbart weiter, dass die reinen Kohlenstoffnanoröhrchen keinen katalytischen Effekt zeigen. Es wird nicht offenbart, dass die verwendeten Kohlenstoffnanoröhrchen durch Heteroatome modifiziert worden sind.

Die Offenbarungen des Standes der Technik bezüglich der Verfahren zur Hydrierung beziehen sich auf die Verwendung rein graphitischen Katalysatormaterialien in Form von Kohlenstoffnanoröhrchen und sind dadurch gekennzeichnet, dass sie auf teure Edelmetalle (bspw. Pt) als katalytisch aktive Komponente nicht verzichten können. Lediglich das Trägermaterial konnte ersetzt werden. Damit sind solche Katalysatoren und Verfahren unter Verwendung dieser, wirtschaftlich nach wie vor unvorteilhaft.

Kohlenstoffnanoröhrchen sind als Modifikation des Kohlenstoffs seit 1991 (S. Iijima, Nature 354, 56-58, 1991) dem Fachmann allgmein bekannt. Unter Kohlenstoffnanoröhrchen werden seither zylinderförmige Körper umfassend Kohlenstoff mit einem Durchmesser zwischen 3 und 80 nm und einer Länge, die ein Vielfaches, mindestens 10-faches, des Durchmessers beträgt, zusammengefasst. Weiterhin kennzeichnend für diese Kohlenstofmanoröhrchen sind Lagen geordneter Kohlenstoffatome, wobei im Regelfall die Kohlenstoffnanoröhrchen einen in der Morphologie unterschiedlichen Kern aufweisen. Synonyme für Kohlenstoffnanoröhrchen sind beispielsweise "carbon fibrils" oder "hollow carbon fibres" oder "carbon bamboos" oder (im Fall von aufgewickelten Strukturen) "Nanoscrolls" oder "Nanorolls".

Es ist weiter bekannt, dass die oben genannten Kohlenstoffnanoröhrchen durch Heteroatome z.B. der fünften Hauptgruppe (wie etwa Stickstoff) während des Verfahrens zu ihrer Herstellung modifiziert werden können.

Van Dommele et al. und Matter et al. (S. van Dommele et al., Stud. Surf. Sci. and Cat., 2006, 162, 29-36, ed.: E.M. Gaigneaux et al.; P.H. Matter et al., J. Mol. Cat A: Chemical 264 (2007), 73-81) offenbaren je eine typische Ausführungsform von Verfahren um solche Stickstoff-dotierten Kohlenstoffnanoröhrchen zu erhalten. Van Dommele et al. offenbaren weiter, dass die erhaltenen, basischen Katalysatoren für Verfahren in der Feinchemie geeignet sein können. Beide offenbaren nicht, dass die offenbarten Katalysatoren in Verfahren zur Hydrierung von organischen Verbindungen verwendet werden können, noch dass besondere Anordnungen des Stickstoffs in solchen Stickstoff-dotierten Kohlenstoffnanoröhrchen besonders vorteilhaft hierfür sein könnten.

In der WO 2005/035841 wird offenbart, dass Katalysatoren umfassend Stickstoff-dotierte Kohlenstoffnanoröhrchen für die Reduktion von Sauerstoff katalytisch aktiv sind. Es wird in diesem Zusammenhang weiter offenbart, dass solche Katalysatoren umfassend Stickstoff-dotierte Kohlenstoffnanoröhrchen eine bessere katalytische Aktivität für die Reduktion von Wasserstoffperoxid besitzen als nicht-dotierte Kohlenstoffnanoröhrchen. Es wird nicht offenbart, dass eine Hydrierung mit den Katalysatoren möglich ist, noch welche besondere Anordnungen des Stickstoffs in solchen Stickstoff-dotierten Kohlenstoffnanoröhrchen besonders vorteilhaft hierfür sein könnten.

Ausgehend vom bekannten Stand der Technik besteht also die Aufgabe ein Verfahren umfassend die Verwendung eines Katalysators zur Verfügung zu stellen, das die Hydrierung von organischen Verbindungen ohne den Einsatz von teuren Edelmetallen als katalytisch aktive Komponente für die Hydrierung ermöglicht. Der Katalysator soll hierbei auch keine pyrophoren katalytisch aktiven Komponenten umfassen, damit das neue Verfahren auf deren Verwendung verzichten kann.

Es wurde überraschend gefunden, dass diese Aufgabe mit den hier offenbarten Katalysatoren und Verfahren unter Verwendung dieser Katalysatoren nun erstmals und in besonders vorteilhafter Weise gelöst werden kann.

Diese Aufgabe wird gelöst durch Bereitstellen eines Verfahrens zur Hydrierung von organischen Verbindungen mit Wasserstoff, dadurch gekennzeichnet, dass die Hydrierung in Gegenwart eines Katalysators durchgeführt wird, der als katalytisch aktive Komponente Stickstoff-dotierte Kohlenstoffnanoröhrchen umfasst, deren Anteil an Stickstoff zwischen 0,05 und 20 Gew.-% in den graphitischen Schichten beträgt und deren Stickstoff mindestens teilweise in pyridinischer Anordnung vorliegt.

Das erfindungsgemäße Verfahren verwendet einen Katalysator zur Hydrierung von organischen Verbindungen, der Stickstoff-dotierte Kohlentoffnanoröhrchen als katalytisch aktive Komponente umfasst und der dadurch gekennzeichnet ist, dass der Anteil an Stickstoff an den Stickstoff-dotierten Kohlenstoffnanoröhrchen zwischen 0,05 und 20 Gew.-% in den graphitischen Schichten beträgt und dass der Stickstoff mindestens teilweise in pyridinischer Anordnung vorliegt.

Der erfindungsgemäße Katalysator umfasst bevorzugt Stickstoff-dotierte Kohlenstoffnanoröhrchen, die einen Stickstoffanteil von 0,1 Gew.-%, bis 18 Gew.-% und besonders bevorzugt von 0,5 Gew.-% bis 16 Gew.-% in den graphitischen Schichten enthalten.

Die Anteile an Stickstoff in den Stickstoff-dotierten Kohlenstoffnanoröhrchen sind besonders vorteilhaft, weil sich überraschend gezeigt hat, dass für die Hydrierung von organischen Verbindungen ein Optimum des Stickstoffanteils an den Stickstoff-dotierten Kohlenstoffnanoröhrchen existiert. Ausgehend vom Stand der Technik hätte es nahegelegen zu vermuten, dass eine weitere Erhöhung des Stickstoffanteils mit einer weiter verbesserten katalytischen Aktivität der Stickstoff-dotierten Kohlenstoffnanoröhrchen einhergehen würde. Im Gegensatz hierzu wurde überraschend das im oben angegebenen Bereich befindliche Optimum gefunden.

Der in den Stickstoff-dotierten Kohlenstoffnanoröhrchen enthaltene Anteil Stickstoff kann neben mindestens einem Anteil pyridinischen Stickstoffs auch in Form von quartenärem Stickstoff und/oder Nitro Stickstoff und/oder Nitroso Stickstoff und/oder Aminem Stickstoff vorliegen.

Die Anteile an Nitro und/oder Nitroso und/oder Aminem Stickstoff sind für die vorliegende Erfindung insofern von nachrangiger Bedeutung, als dass ihr Vorhandensein die Erfindung nicht signifikant behindert, solange die nun beschriebenen Anteile und Verhältnisse zueinander in Bezug auf pyridinischen und/oder quarternären Stickstoff vorhanden sind.

Der Anteil an pyridinischem Stickstoff an dem Katalysator beträgt bevorzugt mindestens 20 mol-% am in den Stickstoff-dotierten Kohlenstoffnanoröhrchen enthaltenen Stickstoff. Besonders bevorzugt ist der Anteil größer als 30 mol-%.

Ebenfalls bevorzugt beträgt das Verhältnis von pyridinischem zu quarternärem Stickstoff mindestens 1,25 mol/mol, besonders bevorzugt beträgt das Verhältnis mindestens 1,3 mol/mol.

Die angegebenen Anteile und Verhältnisse zueinander sind besonders vorteilhaft, weil überraschend gefunden wurde, dass insbesondere der pyridinsche Stickstoff einen besonders starken Einfluss auf die katalytische Aktivität des Katalysators zur Hydrierung von organischen Verbindungen hat.

Ohne den Anspruch einer vollständigen und hierauf beschränkenden Theorie kann vermutet werden, dass die symmetrisch Kohlenstoff-stabilisierte Anordnung des Stickstoffs in pyridinischer Anordnung in besonders vorteilhafter Weise einen stabilisierenden Zwischenkomplex mit Wasserstoff eingeht, der hierdurch als reaktive Spezies besser zur Hydrierung des zu hydrierenden organischen Stoffes zur Verfügung steht. Bei quarternärem Stickstoff könnte man eine sterische Hinderung vermuten, während die oben aufgeführten anderen Erscheinungsformen des Stickstoffs in den Stickstoff-dotierten Kohlenstoffnanoröhrchen nicht symmetrisch stabilisiert sind und hierdurch kein stabilisierender Zwischenkomplex mit dem Wasserstoff entstehen kann.

In einer bevorzugten Variante werden die Stickstoff-dotierten Kohlenstoffnanoröhrchen einer Wäsche mit einer mineralischen oder organischen Säure wie beispielsweise HCl, H₂SO₄ usw. unterzogen.

Eine solche Wäsche ist vorteilhaft, da hierdurch Restbestandteile des Katalysators, mit dem die Stickstoff-dotierten Kohlenstoffnanoröhrchen hergestellt werden, entfernt werden, und hierdurch gegebenenfalls an diesen Resten von Katalysator stattfindende Nebenreaktionen minimiert werden können.

Es ist ebenfalls möglich, dass die Stickstoff-haltigen Kohlenstoffnanoröhrchen chemisch funktionalisiert vorliegen.

Eine chemische Funktionalisierung ist besonders vorteilhaft, da hierdurch gegebenenfalls eine verbesserte Wechselwirkung des zu hydrierenden Substrates mit dem katalytisch aktiven Stickstoff-dotierten Kohlenstoffnanoröhrchen induziert werden kann, d.h. die Oberflächeneigenschaften im Sinne der Hydrierreaktion verbessert werden können.

Üblicherweise beträgt der Anteil der Stickstoff-dotierten Kohlenstoffnanoröhrchen am Katalysator mindestens 50 Gew.-%. Bevorzugt beträgt der Anteil mindestens 80 Gew.-%. Besonders bevorzugt mindestens 95 Gew.-%.

Neben den Stickstoff-dotierten Kohlenstoffnanoröhrchen kann der Katalysator auch noch Füllstoffe oder Spuren von Metallen umfassen.

Füllstoffe bezeichnen im Zusammenhang mit der vorliegenden Erfindung alle Stoffe, die als inertes Material zusammen mit den Stickstoff-dotierten Kohlenstoffnanoröhrchen vorliegen. D.h. alle Stoffe, die nicht katalytisch aktiv sind. Nicht abschließende Beispiele für Füllstoffe sind etwa Alumina, Silica, Titandioxid, Zirkondioxid oder deren Gemischen Steatit, Keramik, Glas oder Graphit.

Spuren von Metallen bezeichnen im Zusammenhang mit der vorliegenden Erfindung einen Anteil von Metallen von weniger als 1 Gew.-% des Katalysators. Mögliche Metalle, die in Spuren vorhanden sein können, sind die Metalle ausgewählt aus der Liste enthaltend Fe, Ni, Cu, W, V, Cr, Sn, Co, Mn und Mo.

Diese Metalle können oder können nicht katalytisch aktiv sein. Die Tatsache, ob die Spuren von Metallen katalytisch aktive Komponenten umfassen, ist nicht wesentlich für die vorliegende Erfindung. Der erfindungsgemäße katalytische Effekt beruht auf den besonderen Stickstoffdotierten Kohlenstoffnanoröhrchen, nicht aber auf den Spuren von Metallen.

In einer bevorzugten Weiterentwicklung des Katalysators werden daher die Spuren der Metalle aus den Stickstoff-dotierten Kohlenstoffnanoröhrchen ausgewaschen, so das der Katalysator keine Spuren von Metallen mehr umfasst.

Ein solches Auswaschen kann in Form eines nasschemischen Reinigungsschritts ausgeführt werden, indem die Stickstoff-dotierten Kohlenstoffnanoröhrchen mit einer mineralischen Säure, bspw. ausgewählt aus der Reihe: HCl, H₂SO₄, HNO₃, HClCO₄ usw. behandelt werden. Bevorzugt wird HCl und HNO₃ eingesetzt, ganz bevorzugt HCl. Die Konzentration der Säure beträgt bevorzugt 0,1 mol/L bis 36 gew.% HCl und ganz bevorzugt 1 mol/L bis 10 mol/L. Der Gegenstand der vorliegenden Erfindung ist ein Verfahren zur Hydrierung von organischen Verbindungen mit Wasserstoff, das dadurch gekennzeichnet ist, dass die Hydrierung in Gegenwart eines Katalysators durchgeführt wird, der als katalytisch aktive Komponente Stickstoff-dotierte Kohlenstoffnanoröhrchen umfasst, deren Anteil an Stickstoff zwischen 0,05 und 20 Gew.-% in den graphitischen Schichten beträgt und deren Stickstoff mindestens teilweise in pyridinischer Anordnung vorliegt.

Organische Verbindungen bezeichnen im Zusammenhang mit der vorliegenden Erfindung alle Stoffe, die mindestens eine hydrieraktive funktionelle Gruppe umfassen. Dem Fachmann ist allgemein bekannt, welche chemischen Gruppen als hydrieraktive funktionelle Gruppen bezeichnet werden können. Nicht abschließende Beispiele hierfür bilden Nitro-Gruppen, C-C-Doppel-bindungen, C-C-Dreifachbindungen, aromatische Ringe, Carbonylgruppen, Nitril-Gruppen, Ester-gruppen, Diolefine, usw.

Das erfindungsgemäße Verfahren kann entweder so durchgeführt werden, dass die organische Verbindung und der Wasserstoff gasförmig vorliegen oder dass die organische Verbindung in einer flüssigen Phase vorliegt und der Wasserstoff gasförmig.

Liegt die organische Verbindung in einer flüssigen Phase vor, so kann die flüssige Phase durch die organische Verbindung selber gebildet wird oder die organische Verbindung in einem flüssigen Lösungsmittel vorliegen.

Liegt die organische Verbindung in einem Lösungsmittel vor, so kann das Lösungsmittel ein organisches Lösungsmittel oder Wasser sein. Bevorzugt ist das Lösungsmittel ein organisches Lösungsmittel. Besonders bevorzugt ist es ein organisches Lösungsmittel, das keine hydrieraktive funktionelle Gruppe umfasst oder ein Gemisch solcher organischer Lösungsmittel.

Nicht abschließend Beispiele für organische Lösungsmittel, die für die alternativ Ausführungsform der Erfindung verwendet werden können, sind unsubstituierte oder substituierte aromatische oder nicht aromatische Kohlenwasserstoffe, mit Allylrest oder Halogen als Substituent. Bevorzugt sind Alkane, Halogenalkane, Alkohole und Ether. Besonders bevorzugt sind Hexan, Methylcyclohexan, Heptan, Methanol, Ethanol und i.-Propanol.

Üblicherweise liegt die organische Verbindung in den Lösungsmitteln in Anteilen von 0,1 Gew.-% bis 60 Gew.-%, bevorzugt 1 Gew.-% bis 55 Gew.-% und besonders bevorzugt von 2,5 Gew.-% bis 50 Gew.-% vor.

Die Temperaturen, bei denen die Hydrierungen bei Vorliegen einer flüssigen Phase umfassend die organische Verbindung, durchgeführt werden, liegen üblicherweise zwischen 20°C und 350°C, bevorzugt im Bereich von 40°C und 300°C.

Die obere Grenze wird durch sicherheitstechnische Gründe vorgegeben. Das erfindungsgemäße Verfahren ist hier eigentlich nicht begrenzt. Die untere Grenze bilden Temperaturen, bei denen trotz der hohen Aktivität des verwendeten Katalysators die geringen Umsätze und Raum-Zeit-Ausbeuten das Verfahren unwirtschaftlich machen.

Der Gesamtdruck für die Durchführung von Hydrierungen bei Vorliegen einer flüssigen Phase, umfassend die organische Verbindung, beträgt üblicherweise 1 bar bis 300 bar, bevorzugt 1 bar bis 200 bar, besonders bevorzugt 1 bar bis 50 bar.

Auch hier definieren sicherheitstechnische Gründe die Obergrenze. Das erfindungsgemäße Verfahren ist hierdurch eigentlich nicht begrenzt. Die untere Grenze bilden Drücke, bei denen trotz der hohen Aktivität des verwendeten Katalysators die geringen Umsätze und Raum-Zeit-Ausbeuten das Verfahren unwirtschaftlich machen.

Der Partialdruckprozentsatz von Wasserstoff bei Hydrierungen unter Vorliegen einer flüssigen Phase umfassend die organische Verbindung, ausgedrückt in einem Prozentsatz des Anteils des Wasserstoffpartialdruckes am Gesamtdruck des Verfahrens, kann zwischen 50 und 100% liegen. Bevorzugt liegt er über 70%. Besonders bevorzugt über 90%.

Die Vorrichtungen, in denen ein solches Verfahren ausgeführt werden kann, sind die dem Fachmann unter den Oberbegriffen allgemein bekannten Reaktionsapparate wie etwa Rührkessel (Slurry-Reaktor), Düsenreaktor, Blasensäule, Rieselbettreaktor, Membranreaktor usw.

Liegt die organische Verbindung in der Gasphase vor, so kann in dieser alternativen Ausführungsform das Verfahren bei Temperaturen im Bereich von 120 bis 750°C, bevorzugt 140 bis 650°C, besonders bevorzugt von 200 bis 600°C durchgeführt werden.

Üblicherweise liegt der absolute Reaktionsdruck bei gasförmigem Vorliegen der organischen Verbindung im Bereich von 1 bis 100 bar, bevorzugt 1,2 bis 75 bar, besonders bevorzugt 1,5 bis 50 bar.

Die Vorrichtungen, in denen ein solches Verfahren ausgeführt werden kann, sind die dem Fachmann unter den Oberbegriffen allgemein bekannten Reaktionsapparate wie etwa Festbett-oder Wirbelbettreaktoren, sowie Rohrbündelreaktoren.

Das erfindungsgemäße Verfahren kann unabhängig von der Tatsache, ob die organische Verbindung in einer flüssigen Phase vorliegt oder gasförmig vorliegt, adiabat oder isotherm oder annähernd isotherm ausgeführt werden. Bevorzugt wird es annähernd isotherm ausgeführt.

Weiter kann es kontinuierlich oder diskontinuierlich ausgeführt werden. Bevorzugt wird es kontinuierlich ausgeführt.

Ebenfalls unabhängig hiervon kann das Verfahren ein- oder auch mehrstufig durchgeführt werden.

In einer bevorzugten Weiterentwicklung des Verfahrens können 2 bis 10, bevorzugt 2 bis 6, besonders bevorzugt 2 bis 5, insbesondere 2 bis 3, in Reihe geschaltete Reaktionszonen vorliegen, zwischen denen gegebenenfalls Abkühlzonen vorgesehen werden können.

Der Wasserstoff kann entweder vollständig zusammen mit der organischen Verbindung vor der ersten Reaktionszone oder über die verschiedenen Reaktionszonen verteilt zugegeben werden. Diese Reihenschaltung einzelner Reaktionszonen kann in einem der zuvor offenbarten Reaktionsapparate zusammengefasst vorliegen oder auf einzelne Reaktionsapparate aufgeteilt sein.

Der im erfindungsgemäßen Verfahren verwendete Katalysator umfasst bevorzugt Stickstoff-dotierte Kohlenstoffnanoröhrchen, die einen Stickstoffanteil von 0,1 Gew.-% bis 20 Gew.-% und besonders bevorzugt von 0,5 Gew.-% bis 18 Gew.-% in den graphitischen Schichten enthalten.

Ebenfalls bevorzugt beträgt der Anteil an pyridinischem Stickstoff am in den Stickstoff-dotierten Kohlenstoffnanoröhrchen enthaltenen Stickstoff mindestens 20 mol-%. Besonders bevorzugt ist der Anteil größer als 30 mol-%.

Weiter bevorzugt beträgt das Verhältnis vom pyridinischen zum quarternären Stickstoff des in den Stickstoff-dotierten Kohlenstoffnanoröhrchen enthaltenen Stickstoff mindestens 1,25 mol/mol, besonders bevorzugt beträgt das Verhältnis mindestens 1,3 mol/mol.

Der Katalysator kann als lose Schüttung oder als Festbett vorliegen. Bevorzugt liegt der Katalysator als Festbett vor.

In einer bevorzugten Weiterentwicklung des erfindungsgemäßen Verfahrens liegt der Katalysator als Festbett in Form einer strukturierten Schüttung vor, bei der die Katalysatoraktivität in Richtung der Hauptströmungsrichtung in der Reaktionszone ansteigt.

Eine solche strukturierte Schüttung kann bevorzugt durch einen unterschiedlichen Gehalt von Stickstoff-dotierten Kohlenstoffnanoröhrchen am erfindungsgemäßen Katalysator oder durch unterschiedliche Verdünnung mit einem Inertmaterial oder durch Verwendung von Katalysatoren, umfassend unterschiedliche Anteile an quarternärem Stickstoff in den Stickstoff-dotierten Kohlenstoffnanoröhrchen, erzielt werden.

In einer weiteren bevorzugten Ausführungsförm des Verfahrens liegt der Katalysator als Formkörper in der Reaktionszone vor. Auch in dieser weiteren bevorzugten Ausführungsform kann eine Verdünnung mit Inertmaterial vorgesehen werden. Wird eine solche Verdünnung mit Inertmaterial vorgesehen, so liegt das Inertmaterial bevorzugt in Form ähnlicher Formkörper wie der Katalysator neben diesen vor.

Formkörper bezeichnen im Zusammenhang mit der vorliegenden Erfindung geometrische Körper aus den oben angegebenen Materialien mit beliebigen, vordefinierten Formen, die z.B. durch Pressen erhalten werden können. Bevorzugte Formkörper sind Tabletten, Ringe, Zylinder, Sterne, Wagenräder oder Kugeln. Besonders bevorzugt sind Kugeln, Ringe, Zylinder oder Sternstränge.

Die Größe dieser Formkörper liegt üblicherweise zwischen 500 µm bis 5 mm und ganz bevorzugt von 1 bis 3 mm.

Für den Fall, dass die organische Verbindung in einer flüssigen Phase vorliegt kann in einer alternativen Ausführungsform des erfindungsgemäßen Verfahrens auch ein feines Pulver des Katalysators eingesetzt werden. Das Pulver wird bevorzugt in einer Partikelgröße von 10-1000 µm bevorzugt 100-900 µm eingesetzt.

Das erfindungsgemäße Verfahren erlaubt in überraschend vorteilhafter Weise die heterogen katalysierte Hydrierung von organischen Verbindungen mit hohem Umsatz, Selektivität und Ausbeute und verzichtet auf die Verwendung teurer Edelmetalle bzw. pyrophorer Katalysatorbestandteile.

Im Folgenden wird das erfindungsgemäße Verfahren anhand von Beispielen näher erläutert, wobei die Beispiele jedoch nicht als Einschränkung des Erfindungsgedankens zu verstehen sind.

### Beispiele:

### Beispiel 1: Herstellung von Stickstoff-dotierten Kohlenstoffnanoröhrchen

72 g eines Co-Mn-Al-Mg-Mischoxid Katalysators (Mn:Co:Al₂O₃:MgO 17:18:44:22), hergestellt wie beispielsweise in WO 2007/093337, Beispiel 2 beschrieben, wurden in einen Wirbelschichtreaktor gegeben, in dem sich bereits eine Vorlage aus 350 g herkömmlicher Kohlenstoffnanoröhrchen-Agglomerate (Baytubes®, Fa. Bayer MaterialScience AG) befand, um eine gleichmäßige Verteilung des Gasstromes und der Temperatur in der Wirbelschicht zu erzielen.

Die Partikeln des Co-Mn-Al-Mg-Mischoxid Katalysators hatten einen Durchmesser zwischen 32 µm und 90 µm. Der Reaktor wurde von außen elektrisch auf eine Reaktionstemperatur von 750°C beheizt und nach Durchleiten von Stickstoff (Interisieren) wurde das Reaktionsgemisch, bestehend aus 15 g/min Acetonitril, 25 LN/min Stickstoff und 3,6 LN/min Wasserstoff, durch einen Lochboden am unteren Ende des Reaktors in den Wirbelschichtreaktor geleitet.

Die Herstellung des Eduktgasgemisches erfolgte in einem vorgeschalteten elektrisch beheizten Festbett (Durchmesser 50 mm, Höhe 1.000 mm, Füllung mit Glas-Raschigringen). In dieses wurde das Acetonitril mittels einer Dosierpumpe flüssig eindosiert; zum verdampfenden Acetonitril wurde der Stickstoff und der Wasserstoff gasförmig zudosiert, so dass aus dem Festbett ein überhitztes Gasgemisch mit einer Temperatur von 200°C in den Wirbelschichtreaktor austritt.

In der Wirbelschicht bildeten sich am Co-Mn-Al-Mg-Mischoxid Katalysator Stickstoff-dotierte Kohlenstoffnanoröhrchen, wodurch die Katalysatorpartikeln gesprengt wurden und die Agglomeratpartikeln aus Stickstoff-dotierten Kohlenstoffnanoröhrchen und Katalysatorresten entstanden.

Die Beaufschlagung des Katalysators mit den Eduktgasen erfolgte über einen Zeitraum von 90 Minuten bis zur vollständigen Desaktivierung des Katalysators. Die Aktivität des Katalysators wurde über die mittels Gaschromatographie bestimmte Wasserstoffentwicklung im Reaktor verfolgt. Als Zeitpunkt der vollständigen Desaktivierung wurde der Zeitpunkt angenommen, bei dem die Wasserstoffentwicklung im Verfahren um mindestens einen Faktor 10 gegenüber dem zunächst gemessenen Wert verringert war.

Nach erneutem Inertisieren wurden dem Reaktor 220 g eines schwarzen Pulvers entnommen, weitere ca. 350 g Produkt verbleiben im Reaktor als Bettvorlage für die nächste Charge. Dieser Vorgang wird 3 mal wiederholt, bis das ganze Kohlenstoffnanoröhrchenbett ersetzt worden ist. Die Menge an Kohlenstoff, enthalten in den hergestellten Stickstoff-dotierten Kohlenstoffnanoröhrchen, in Bezug auf die eingesetzte Masse Katalysator, wird im Weiteren als Ertrag bezeichnet.

Es wurde ein Ertrag von 7,6 g Stickstoff-dotierter Kohlenstoffhanoröhrchen / g Kat erzielt.

Die so hergestellten Stickstoff-dotierten Kohlenstoffnanoröhrchen wurden abschließend 3 h unter Rückfluss mit 2 mol/L HCl gewaschen.

Der in den Stickstoff-dotierten Kohlenstoffnanoröhrchen enthaltene Stickstoff wurde mittels Elementanalyse (Gerät Trustec, Fa. LECO, Methode nach Angaben des Herstellers) bestimmt. Der Stickstoffanteil betrug 5,0 Gew.-%.

Die unterschiedlichen Bindungszustände des Stickstoffes sowie die Quantifizierung der dieser wurden mittels ESCA-Analyse (Firma ThermoFisher, ESCALab 220iXL; Methode nach Angaben des Herstellers) bestimmt. Das Verhältnis von pyridinschem zu quartenärem Stickstoff der erhaltenen Stickstoff-dotierten Kohlenstoffnanoröhrchen betrug 0,81 mol/mol.

### Beispiel 2: Herstellung von Stickstoff-dotierten Kohlenstoffnanoröhrchen

Die Herstellung wurde entsprechend Beispiel 1 durchgeführt, wobei Acetonitril mit einer Dosierrate von 19 g/min in dem Reaktor dosiert wurde. Der Ertrag an Stickstoff-dotierten Kohlenstoffnanoröhrchen betrug 5,9 g / g Katalysator.

Die so hergestellten Stickstoff-dotierten Kohlenstoffnanoröhrchen hatten einen Sickstoffgehalt von 4,1 Gew.-% und ein Verhältnis von pyridinischem zu quartenärem Stickstoff von 1,31 mol/mol.

### Beispiel 3: Herstellung von Stickstoff-dotierten Kohlenstoffnanoröhrchen

Die Herstellung wurde entsprechend Beispiel 1 durchgeführt, wobei die Einwaage an Katalysator 48 g betrug und anstelle Acetonitril Pyridin mit einer Dosierrate von 26 g/min in den Reaktor dosiert wurde. Der Ertrag an Stickstoff-dotierten Kohlenstoffnanoröhrchen betrug 10,1 g / g Katalysator.

Die so hergestellten Stickstoff-dotierten Kohlenstoffnanoröhrchen hatten einen Stickstoffgehalt von 4,4 Gew.-% und ein Verhältnis von pyridinischem zu quartenärem Stickstoff von 1,15 mol/mol.

### Beispiel 4: Herstellung von Stickstoff-dotierten Kohlenstoffnanoröhrchen

Die Herstellung wurde entsprechend Beispiel 3 durchgeführt, wobei die Einwaage an Katalysator 104 g betrug. Der Ertrag an Stickstoff-dotierten Kohlenstoffnanoröhrchen betrug 13,8 g / g Katalysator.

Die so hergestellten Stickstoff-dotierten Kohlenstoffnanoröhrchen hatten einen Stickstoffgehalt von 7,7 Gew.-% und ein Verhältnis von pyridinischem zu quartenärem Stickstoff von 0,59.

### Beispiel 5: Herstellung von Stickstoff-dotierten Kohlenstoffnanoröhrchen

Die Herstellung wurde entsprechend Beispiel 3 durchgeführt, wobei die Einwaage an Katalysator 80 g betrug und anstelle Pyridin Dimetyhlformamid mit einer Dosierrate von 31,5 g/min und kein Wasserstoff in den Reaktor dosiert wurde.

Der Ertrag an Stickstoff-dotierten Kohlenstoffnanoröhrchen betrug 4,2 g / g Katalysator. Die so hergestellten Stickstoff-dotierten Kohlenstoffnanoröhrchen hatten einen Stickstoffgehalt von 3,1 Gew.-% und ein Verhältnis von pyridinischem zu quartenärem Stickstoff von 1,39 mol/mol.

### Beispiel 6: Herstellung von Stickstoff-dotierten Kohlenstoffnanoröhrchen

Die Herstellung wurde entsprechend Beispiel 5 durchgeführt, wobei die Dosierrate zu 10,1 g/min eingestellt wurde. Der Ertrag an Stickstoff-dotierten Kohlenstoffnanoröhrchen betrug 5,0 g / g Katalysator. Die so hergestellten Stickstoff-dotierten Kohlenstoffnanoröhrchen hatten einen Stickstoffgehalt von 3,5 Gew.-% und ein Verhältnis von pyridinischem zu quartenärem Stickstoff von 1,52 mol/mol.

**Tabelle 1: Zusammensetzung und Eigenschaften der Katalysatoren gemäß der Beispiele 1-6**

| **Bsp. [-]** | **Stickstoffgehalt [Gew.%]** | **Pyr./Quart. [mol/mol]** |
|---|---|---|
| 1 | 5,0% | 0,81 |
| 2 | 4,1% | 1,31 |
| 3 | 4,4% | 1,15 |
| 4 | 7,7% | 0,59 |
| 5 | 3,1% | 1,39 |
| 6 | 3,5% | 1,52 |

### Beispiele 7-13: Hydrierung von Nitrobenzol

In einem gerührten Druckreaktor wurden 5 g eines Reaktionsgemisches einer 30%-igen Lösung von Nitrobenzol in Isopropanol vorgelegt. Hierzu wurden jeweils soviel der Stickstoff-dotierten Kohlenstoffnanoröhrchen der Beispiele 1-6, oder von undotierten Kohlenstoffnanoröhrchen (Baytubes®, Fa Bayer MaterialScience AG) zugegeben, so dass diese 3 Gew.-% der Suspension betrugen.

Die Hydrierung wurde stets bei 150°C, 40 bar und einer Rührerumdrehung von 1000 U/min im oben angegebenen Druckreaktor durchgeführt.

Nach 120 min wurde die Reaktion durch Abkühlen des Druckreaktors auf Raumtemperatur (23°C) abgebrochen und das Reaktionsgemisch wurde nach Abtrennen der Stickstoff-dotierten Kohlenstoffnanoröhrchen einer gaschromatographischen Analyse unterzogen (Gerät: Hewlett Packard HP6890, Säule DB-5, 30 m; nach Angaben des Herstellers). Die Anteile an Anilin an den jeweils analysierten Reaktionsgemischen sind in Tablle 2 für die verschiedenen Katalysatoren gemäß der Beispiele 1-6, sowie für die undotierten Kohlenstoffnanoröhrchen zusammengefasst.

**Tabelle 2: Zusammensetzung der Reaktionsgemische und Katalysatoren gemäß der Beispiele 7-13**

| **Bsp.[-]** | **Katalysator** | **Anilin [FI%]** |
|---|---|---|
| 7 | Gemäß Bsp. 1 | 31 |
| 8 | Gemäß Bsp. 2 | 79 |
| 9 | Gemäß Bsp. 3 | 65 |
| 10 | Gemäß Bsp. 4 | 19 |
| 11 | Gemäß Bsp. 5 | 98 |
| 12 | GemäB Bsp. 6 | 100 |
| - | Baytubes® | 0,4 |

Es zeigte sich, dass insbesondere die Stickstoff-dotierten Kohlenstoffnanoröhrchen, die ein Verhältnis von pyridinischem zu quartenärem Stickstoff von größer als 1 eine besonders hohe katalytische Aktivität aufwiesen und demzufolge einen besonders hohen Anteil von Anilin im Reaktionsprodukt nach Umsatz im erfindungsgemäßen Verfahren aufwiesen.

Es zeigte sich außerdem eine überraschenderweise beinahe lineare Korrelation zwischen dem Verhältnis von pyridinischem zu quarternären Stickstoff und dem erzielbaren Umsatz zu Anilin.

## Patentansprüche

1. Verfahren zur Hydrierung von organischen Verbindungen mit Wasserstoff, **dadurch gekennzeichnet, dass** die Hydrierung in Gegenwart eines Katalysators durchgeführt wird, der als katalytisch aktive Komponente Stickstoff-dotierte Kohlenstoffnanoröhrchen umfasst, deren Anteil an Stickstoff zwischen 0,05 und 20 Gew.-% in den graphitischen Schichten beträgt und deren Stickstoff mindestens teilweise in pyridinischer Anordnung vorliegt.

2. Verfahren nach Anspruch 1, **dadurch gekennzeichnet, dass** die organische Verbindung in einer flüssigen Phase vorliegt und der Wasserstoff gasförmig vorliegt.

3. Verfahren nach Anspruch 2, **dadurch gekennzeichnet, dass** die organische Verbindung in einem flüssigen Lösungsmittel vorliegt.

4. Verfahren nach Anspruch 3, **dadurch gekennzeichnet, dass** das Lösungsmittel ein organisches Lösungsmittel ist, das keine hydrieraktive funktionelle Gruppe umfasst, oder ein Gemisch solcher organischer Lösungsmittel ist.

5. Verfahren nach einem der Ansprüche 2 bis 4, **dadurch gekennzeichnet, dass** es bei Temperaturen zwischen 20°C und 350°C bevorzugt im von 40°C und 300°C durchgeführt wird.

6. Verfahren nach Anspruch 1, **dadurch gekennzeichnet, dass** die organische Verbindung und der Wasserstoff gasförmig vorliegen.

7. Verfahren nach Anspruch 1, **dadurch gekennzeichnet, dass** es bei Temperaturen von 120 bis 750°C, bevorzugt 140 bis 650°C, besonders bevorzugt von 200 bis 600°C ausgeführt wird.

8. Verfahren nach einem der Ansprüche 1 bis 7, **dadurch gekennzeichnet, dass** es mehrstufig, bevorzugt in 2 bis 10, besonders bevorzugt in 2 bis 6, ganz besonders bevorzugt 2 bis 5, insbesondere bevorzugt 2 bis 3 in Reihe geschalteten Reaktionszonen ausgeführt wird.

9. Verfahren nach einem der Ansprüche 1 bis 8, **dadurch gekennzeichnet, dass** der Katalysator mindestens 50 Gew.-%, bevorzugt mindestens 80 Gew.-%, besonders bevorzugt mindestens 95 Gew.-% Stickstoff-dotierter Kohlenstoffnanoröhrchen enthält.

10. Verfahren nach einem der der Ansprüche 1 bis 9, **dadurch gekennzeichnet, dass** der Katalysator als Festbett vorliegt.

11. Verfahren nach Anspruch 10, **dadurch gekennzeichnet, dass** das Festbett Form einer strukturierten Schüttung vorliegt, bei der die Katalysatoraktivität in Richtung der Hauptströmungsrichtung in der Reaktionszone ansteigt.

## Claims

1. Process for hydrogenating organic compounds by means of hydrogen, **characterized in that** the hydrogenation is carried out in the presence of a catalyst which comprises nitrogen-doped carbon nanotubes as catalytically active component, the proportion of nitrogen in the nitrogen-doped carbon nanotubes is in the range from 0.05 to 20% by weight in the graphitic layers and the nitrogen is present at least partly in a pyridinic arrangement.

2. Process according to Claim 1, **characterized in that** the organic compound is present in a liquid phase and the hydrogen in the present in gaseous form.

3. Process according to Claim 2, **characterized in that** the organic compound is present in a liquid solvent.

4. Process according to Claim 3, **characterized in that** the solvent is an organic solvent which does not comprise any hydrogenation-active functional group or is a mixture of such organic solvents.

5. Process according to any of Claims 2 to 4, **characterized in that** it is carried out at temperatures in the range from 20°C to 350°C, preferably from 40°C to 300°C.

6. Process according to Claim 1, **characterized in that** the organic compound and the hydrogen are present in gaseous form.

7. Process according to Claim 1, **characterized in that** it is carried out at temperatures of from 120 to 750°C, preferably from 140 to 650°C, particularly preferably from 200 to 600°C.

8. Process according to any of Claims 1 to 7, **characterized in that** it is carried out in a plurality of stages, preferably in from 2 to 10, particularly preferably from 2 to 6, very particularly preferably from 2 to 5, in particular 2 or 3, reaction zones connected in series.

9. Process according to any of Claims 1 to 8, **characterized in that** the catalyst contains at least 50% by weight, preferably at least 80% by weight, particularly preferably at least 95% by weight, of nitrogen-doped carbon nanotubes.

10. Process according to any of Claims 1 to 9, **characterized in that** the catalyst is present as a fixed bed.

11. Process according to Claim 10, **characterized in that** the fixed bed is present in the form of a structured bed in which the catalyst activity increases in the main flow direction in the reaction zone.

## Revendications

1. Procédé pour l'hydrogénation de composés organiques avec de l'hydrogène, **caractérisé en ce qu'**on effectue l'hydrogénation en présence d'un catalyseur qui comprend comme composant catalytiquement actif des nanotubes de carbone dopés à l'azote, dont la teneur en azote est comprise entre 0,05 et 20 % en poids dans les couches graphitiques et dont l'azote se trouve au moins en partie en configuration pyridinique.

2. Procédé selon la revendication 1, **caractérisé en ce que** le composé organique est présent dans une phase liquide et l'hydrogène est présent à l'état gazeux.

3. Procédé selon la revendication 2, **caractérisé en ce que** le composé organique est présent dans un solvant liquide.

4. Procédé selon la revendication 3, **caractérisé en ce que** le solvant est un solvant organique qui ne comporte aucun groupe fonctionnel actif dans l'hydrogénation, ou un mélange de tels solvants organiques.

5. Procédé selon l'une quelconque des revendications 2 à 4, **caractérisé en ce qu'**il est effectué à des températures comprises entre 20 °C et 350 °C, de préférence dans la plage de 40 °C à 300 °C.

6. Procédé selon la revendication 1, **caractérisé en ce que** le composé organique et l'hydrogène se trouvent à l'état gazeux.

7. Procédé selon la revendication 1, **caractérisé en ce qu'**il est effectué à des températures de 120 à 750 °C, de préférence de 140 à 650 °C, de façon particulièrement préférée de 200 à 600 °C.

8. Procédé selon l'une quelconque des revendications 1 à 7, **caractérisé en ce qu'**il est effectué en plusieurs stades, de préférence dans 2 à 10, de façon particulièrement préférée dans 2 à 6, de façon tout particulièrement préférée 2 à 5, de façon plus particulièrement préférée 2 à 3 zones de réaction raccordées en série.

9. Procédé selon l'une quelconque des revendications 1 à 8, **caractérisé en ce que** le catalyseur contient au moins 50 % en poids, de préférence au moins 80 % en poids, de façon particulièrement préférée au moins 95 % en poids de nanotubes de carbone dopés à l'azote.

10. Procédé selon l'une quelconque des revendications 1 à 9, caractérisé en ce le catalyseur se trouve sous forme de lit fixe.

11. Procédé selon la revendication 10, **caractérisé en ce que** le lit fixe se trouve sous forme d'un lit fixe dans lequel l'activité du catalyseur augmente dans la direction du dens d'écoulement principal dans la zone de réaction.
